Europäisches Patentamt

European Patent Office

Office européen des brevets

(11) Publication number: **0 082 862**
**B1**

# (12) EUROPEAN PATENT SPECIFICATION

(45) Date of publication of patent specification: **29.01.86**

(51) Int. Cl.⁴: **G 01 N 33/50, B 01 D 39/06**

(21) Application number: **82902066.8**

(22) Date of filing: **06.07.82**

(86) International application number:
**PCT/DK82/00064**

(87) International publication number:
**WO 83/00229 20.01.83 Gazette 83/02**

(54) **A METHOD OF DETECTING OR DETERMINING HISTAMINE IN HISTAMINE CONTAINING MATERIALS, PARTICULARLY BODY FLUIDS AND AN ANALYTICAL MEANS FOR USE IN SUCH METHOD.**

(30) Priority: **06.07.81 DK 2982/81**

(43) Date of publication of application:
**06.07.83 Bulletin 83/27**

(45) Publication of the grant of the patent:
**29.01.86 Bulletin 86/05**

(84) Designated Contracting States:
**AT BE CH DE FR GB LI LU NL SE**

(56) References cited:
**EP-A-0 045 476**
**DE-A-2 922 958**
**GB-A-1 242 493**
**GB-A-2 053 022**
**US-A-4 020 151**

**Chemical Abstract, Vol 67 (1967) abstract no 62 688, Med. Pharmacol. Exp. 16(5), 459-61 (1967) Chemical Abstract, vol 80 (1974), abstract No 24 425, Lab Delo 1973, (9), 543-6**

(73) Proprietor: **SKOV, Per Stahl**
**Kanslergade 6 st. th.**
**DK-2100 Copenhagen O (DK)**
(73) Proprietor: **NORN, Svend**
**Skovvang 1**
**DK-3460 Birkerod (DK)**
(73) Proprietor: **WEEKE, Bent**
**Tollosevej 20**
**DK-2700 Bronshoj (DK)**

(72) Inventor: **SKOV, Per Stahl**
**Kanslergade 6 st. th.**
**DK-2100 Copenhagen O (DK)**
Inventor: **NORN, Svend**
**Skovvang 1**
**DK-3460 Birkerod (DK)**
Inventor: **WEEKE, Bent**
**Tollosevej 20**
**DK-2700 Bronshoj (DK)**

(74) Representative: **Collier, Jeremy Austin Grey et al**
**J.A.Kemp & Co. 14, South Square Gray's Inn**
**London WC1R 5EU (GB)**

EP 0 082 862 B1

Courier Press, Leamington Spa, England.

# 0 082 862

(56) References cited:

Chemical Abstract, Vol 80 (1974), abstract No
67 934, Vop. Med. Khim. 1973 19(6), 655-9
J.Bosse and O. Wassermann, Med. Pharmacol.
Exp. 16(5) 459-461 (1967)
J. Murray & A.S. McGill, I. Assoc. Off. Anal.
Chem. Vol. 65, No. 1 (1982)
"Glass Microfibre Filters", Pub. No. 603,
Whatman, Springfield Mill, Maidstone, Kent.

The file contains technical information
submitted after the application was filed and
not included in this specification

**Description**

The present invention relates to a method of detecting or determining histamine in histamine-containing materials, particularly blood or blood fractions. The invention also relates to an analytical means to be used in the performance of the method.

One of the problems in the treatment of allergic diseases is the lack of diagnostic techniques which are sufficiently specific and sensitive and which do not imply any risk to the patient. Furthermore, there is a need for a rapid, simple, and inexpensive diagnostic technique which would ensure a decrease in the number of patients now waiting for adequate treatment.

In human blood and tissue there exist specific cells (basophil leucocytes and mast cells) which are involved in the allergic reaction. On the surface of these cells is a distinct class of antibodies (IgE-antibodies). When e.g. a patient is allergic to cat, an allergic reaction is caused by the antibodies on the surface of these cells which specifically recognize the protein structure of cat dandruff. When inhaled, cat protein comes in contact with the mast cells and the basophil leucocytes of the lung tissue. The antibodies on the cell surface will react with cat protein, thereby triggering a reaction in the cell, which in turn causes the release of a number of substances (allergic mediators). These allergic mediators are responsible for the symptoms of the patient.

For many years it has been possible to imitate the allergic reaction *in vitro.* This is done by exposing a blood sample from the allergic patient to e.g. cat protein. Allergic mediators are released from the basophil leucocytes in the blood sample. Of these mediators it is possible to determine one substance, i.e. histamine. Therefore, if the patient is allergic to cat, it is possible to determine the release of histamine from the cells. Methods based on this principle represent the best assay for obtaining a correct diagnosis, and are explained in detail below.

By means of histamine determination it is possible to diagnose the responsible antigens (e.g. grass pollen, animal dandruff, drugs, foodstuffs, mould fungi, and bacteria) in patients with allergic diseases (asthma, urticaria, and hay fever).

A general problem of this test is that it is difficult to perform in the laboratory. Few tests can be performed daily and the demand for laboratory technicians is high. Therefore, a simplification of the test is greatly needed so that it could be introduced in the daily diagnostic routine of the clinic.

As mentioned, a number of assays exist for detecting histamine in liquids. Thus, the original histamine determination was described by Shore et al. and was based on a fluorometric assay (ref. 1).

The principle of this assay is a coupling of histamine to a fluorophore (o-phthalaldehyde), whereby a ring structure is formed. The concentration of this ring structure, which can be determined spectrophotometrically, depends on the amount of histamine present. The method was later modified to increase specificity and sensitivity. Stahl Skov & Norn (ref. 2) have thus simplified the assay by allergen-provocation of Ficoll-Hypaque-isolated cell suspensions containing 0.5—2% basophil leucocytes instead of whole blood. By fractionation of the blood, interfering substances are removed, so that the histamine content of the basophil leucocytes can be determined directly, avoiding a long extraction procedure. However, the fractionation procedure (gradient centrifugation) necessary to remove interfering substances is time consuming and difficult to perform, and Siraganian therefore developed an autoanalytical fluorometric method (ref. 3). This method is used in the clinic, but has only found limited application, due to its demand for technical experience, constant monitoring, and expensive apparatus.

Taylor et al. (ref. 4) has developed another assay for determining histamine in biological material. This very sensitive and specific method is an enzymatic isotope technique based on methylation of histamine by means of N-methyltransferase. This assay is useful in determining small amounts of histamine in tissue, blood and urine. However, the need for a routine method to be used in the clinic is not satisfied by this method since the number of assays that can be performed in a day is low (approx. 30) and the assay time is long.

Stahl Skov et al. (ref. 5) has developed another method based on in vitro incorporation of radioactively labelled histamine in the basophil cells of the patient, where the release of labelled histamine is determined after provocation with the suspected allergens.

However, as illustrated below, a poor correlation with the release of histamine determined fluorometrically was obtained by this method.

The purpose of the present invention is to provide a method for the detection or determination of histamine, which is not vitiated by the drawbacks of the known methods such as apparatus requirements, time consumption, and pretreatment of blood samples.

In particular, it is the purpose of the present invention to provide a simple and specific method for the detection and determination of histamine in histamine-containing materials, which is rapid and easy to perform, which requires only a small amount of material, and which does not make heavy demands on the training of personnel.

According to the present invention, a method for detecting or determining histamine in a body fluid, in which a sample of the body fluid is contacted with a binder for histamine and the amount bound is registered or measured, is characterized by contacting the sample of fluid and optionally a predetermined amount of labelled histamine with borosilicate glass microfibres deposited onto a carrier in such a quantitative proportion between the glass microfibres and the fluid as will permit the histamine present to be bound to the microfibres; and registering or measuring the amount of histamine bound to the microfibres, without a previous desorption, either by a competitive determination based on a standard curve for known amounts of unlabelled histamine or by a direct determination. As mentioned, the invention also provides a means for use in this method, comprising borosilicate glass microfibres deposited onto a carrier.

The process of the invention utilizes the finding that histamine is firmly and rather selectively bound to borosilicate glass microfibres even after repeated washings. Such microfibres are extensively used as filters and described in detail, e.g. in the brochure "Glass Microfibre Filters", Publication No. 603, Whatman, Springfield Mill, Maidstone, Kent, England. In this brochure it is emphasised that the microfibres exhibit extremely low adsorption capacity, but have in a few cases been used as a means to adsorb high molecular substances, especially proteins, such as albumin and poly-U in RNA assays. No reference to histamine adsorption is given.

The adsorption of histamine to glass surfaces has been examined by Bosse & Wassermann (Ref. 6). Having recognized certain inaccuracies in the well-known cumbersome detection of histamine by measuring the contraction response of the guinea pig ileum they investigated whether the inaccuracies could be due to a partial binding of histamine to glass surfaces of the test equipment.

To this end they added a coarse powder of various kinds of glass to histamine solutions of various known concentrations. After shaking, the free residual histamine content in the solutions was measured by the guinea pig ileum test in comparison with test solutions without glass powder. No investigations of a possible release of the bound histamine from the glass surface was made.

Bosse & Wassermann found that at low histamine concentrations ($10^{-6}$ M) about 75% of the histamine present was bound to the glass powder. Based on this result they concluded that, in quantitative studies with histamine (concentration-response curves), it is to be considered that in lower concentrations ($<10^{-6}$ M) a large share of histamine is lost by adsorption on glass surfaces.

Certainly, this negative statement would not tempt a person skilled in the art of histamine determination to utilize the recognized undesirable binding properties in a quantitative and reproducible method of detecting or determining histamine. On the contrary the established prejudice would teach the skilled person to avoid contact with any glass surface whatsoever, in particular at low histamine concentrations such as those resulting from allergic reactions.

This view is consistent with the conclusions of Murray & McGill (Ref. 7), 1982. Having verified the above-mentioned findings of Bosse et al., Murray & McGill conclude that high sensitive analytical techniques such as the OPT method for histamine would carry a potentially greater risk of error, if the adsorption effect should operate.

Murray & McGill therefore recommend the use of polyethylene containers and acidification, if glass is used, since this prevents adsorption.

One of the advantages of the use of glass microfibres in the present invention is that low molecular weight compounds, such as serotonin and histadine as well as the histamine metabolites 1,4- and 1,5-methyl imidazole acetic acid, do not bind to the fibres, which is demonstrated below.

Borosilicate glass microfibres are commercially available and are non-toxic and not dangerous to use. Borosilicate glass microfibres taken from glass microfibre filters marketed under the trade mark "Whatman® GF/B" have been found to be very suitable for the performance of the present assay. Fibres from "Whatman® GF/C" glass microfibre filters may be used, but have more variable binding properties.

The above-mentioned "Whatman" brochure states that the said fibres are borosilicate fibres with the following typical oxide content:

| | % | | % |
|---|---|---|---|
| $SiO_2$ | 57.9 | CaO | 2.6 |
| $B_2O_3$ | 10.7 | MgO | 0.4 |
| $Fe_2O_3$ | 5.9 | BaO | 5.0 |
| $Al_2O_3$ }<br>$Na_2O$ } | 10.1 | ZnO | 3.9 |
| | | F | 0.6 |
| $K_2O$ | 2.9 | | |

The invention is of course not restricted to the use of borosilicate glass microfibres of the above-mentioned types, and the skilled person will be able to find fibres of optimal binding properties among the commercially available borosilicate glass fibre types by simple binding tests, as described below in example 1.

However, it is believed at present that the most suitable glass microfibres are those derived from "Whatman® GF/B" glass microfibre filters and fibres of similar properties, particularly binding properties. To obtain the desired sensitivity, the glass microfibres are advantageously divided into fine particles as small as 2 to 20 µm, before they are used in the method of the invention. Here too, the most appropriate fibre dimension and degree of disintegration can be determined by tests and also depend on the carrier onto which the fibres are to be deposited to provide the most expedient assay. Another factor to be considered in selecting fibre dimension is the type of sample material to be tested.

The quantitative proportion between the glass microfibres and the histamine-containing material can likewise be determined by tests depending upon the amount of histamine expected to be bound to the fibres. The amount of histamine released at various degrees of allergy is well-known from the literature, so that standard curves for predetermined histamine amounts may be plotted and serve as a basis for determination of the amount of histamine in an unknown sample, e.g. by a competitive determination, as explained in detail below.

A suitable carrier type is a tube preferably of plastic in the form of a small test tube, but microtiter plates, foils or strips may also be used. For allergy diagnostics, the glass fibres may advantageously be deposited onto suitable carriers such as microtiter plates having a suitable number of wells, optionally together with test allergens and made up into suitable test devices, e.g. in the form of diagnostic kits.

The method of the invention is particularly useful for the detection or determination of histamine released as a consequence of an allergic reaction, in which a blood sample is to be used as the test material. In addition to the general advantages explained in the foregoing, this aspect of the method provides a significant advantage over the known methods in that in order to remove interfering substances, e.g. spermidine, from the blood sample, it is sufficient to remove the blood plasma, e.g. by centrifugation and washing with a physiological buffer. If desired, the red blood cells (erythrocytes) may also be removed, which can be done simply, e.g. by sedimentation with the addition of dextran, before the centrifugation for removing the plasma.

The method can also be used for detection or determination of histamine in other body fluids from humans and animals, such as lymph, cerebrospinal fluids or urine, or in tissue samples or tissue extracts. In this context it is noted that histamine is released in diseases other than allergies, e.g. mastocytosis.

As a general measure interfering substances which might be present depending on the material to be analysed for histamine content should be removed before contacting the sample with the glass microfibres.

The registration or measurement of the histamine bound to the microfibres may be determined by two different principles.

1. Competitive determination including the use of labelled histamine.
2. Direct determination.

1. Competitive determination

This principle of registration is based on a competition for the binding sites on the glass microfibres between histamine possibly present in the sample and a predetermined amount of added labelled histamine.

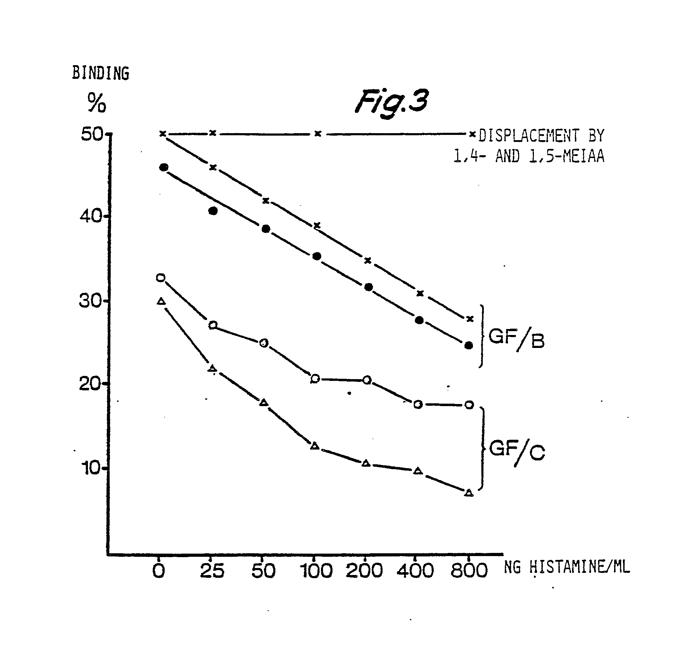

When plotting a standard curve for varying known concentrations of unlabelled histamine together with a given amount of labelled histamine, it is easy to determine the amount of histamine in a specific sample by adding the amount of labelled histamine used for the standard curve. This is explained more fully below in Example 1 with reference to Fig. 3.

In the following Examples 1 and 2 a radioactive isotope ($^3$H-tritium) is used for the labelling of histamine, but other radioactive isotopes may also be used, such as $^{125}$I. The bound amount of radioactively labelled histamine may be easily determined in a manner known per se, e.g. with a scintillation detection counter.

Due to the difficulty in handling radioactive materials, many new test systems have been developed in the allergy diagnostics area which comprise the use of materials other than radioactive isotopes as labelling agents. Examples of these are free radicals, fluorescent molecules (e.g. fluorescein isothiocyanate and rhodamine colouring substances), luminiscent molecules, bacteriophages, enzymes, coenzymes, and enzyme inhibitors.

It generally applies that the labelling agent is not critical, provided that the binding properties of the histamine to the fibres are not affected in a non-reproducible manner, and can be imparted to the histamine according to methods known per se. The crux of the method of the invention is the utilization of the surprisingly selective binding of histamine to the glass microfibres.

When it has been realized in the light of this discovery that a competitive assay is possible and appropriate, it should be a matter of routine for the skilled person to test the conventional labelling methods to find the most suitable methods for the assay in question. As a well-functioning example the use of isotope-labelled histamine in the form of 2,5-$^3$H-histamine dihydrochloride is mentioned, because this procedure is rapid and convenient and provides good accuracy and reproducibility.

2. Direct determination

The selective adsorption properties of the glass microfibres make them suitable for direct binding of the histamine present and for subsequent determination of the bound histamine in a conventional manner, e.g. by coupling with a fluorophore compound and subsequent fluorometric measurement. This procedure is illustrated in Examples 3 and 4 below.

The preparation of plastic tubes containing glass microfibres for use in the present method is easy and inexpensive (5000 to 10,000 tubes per day). The method has proved to be time-saving, as one laboratory technician can prepare about 400 samples per day, as compared with the previous maximum of 150. The method is also blood-saving, as it only requires 10 ml of blood for the determination of 8 to 10 allergies per patient, whereas the known methods require 50 ml of blood.

Since the method is both reproducible and specific and exhibits good correlation with fluorometric histamine determination, it is particularly useful in providing precise information about suspected allergens such as house dust, animal dandruff, pollen, mould fungi, drugs, foodstuffs, bacteria, and autoantigens. The method of the invention is illustrated in detail in the following examples with reference to the drawings, in which

Fig. 1 shows a correlation between histamine determinations performed according to Stahl Skov et al. (Ref. 5) and by fluorometric assay,

Fig. 2 shows the percentage of binding of tritiated histamine, histidine, and serotonin, respectively, in equimolar concentrations to glass microfibres,

Fig. 3 shows the binding of varying concentrations of tritiated histamine to two different glass microfibre types after addition of known amounts of unlabelled histamine and demonstrates the lack of displacement of tritiated histamine caused by the two histamine metabolites 1,4- and 1,5-methyl imidazole acetic acid (MEIAA) and is explained in connection with Example 1,

Fig. 4 shows a correlation between histamine determinations performed by the method of the present invention and by fluorometric assay, and is explained in connection with Example 2, and

Fig. 5 shows an adapted direct fluorometric histamine determination by the method of the present invention with varying washing procedures compared with conventional determination in the absence of fibres (Ref. 2), and is explained in connection with Example 3.

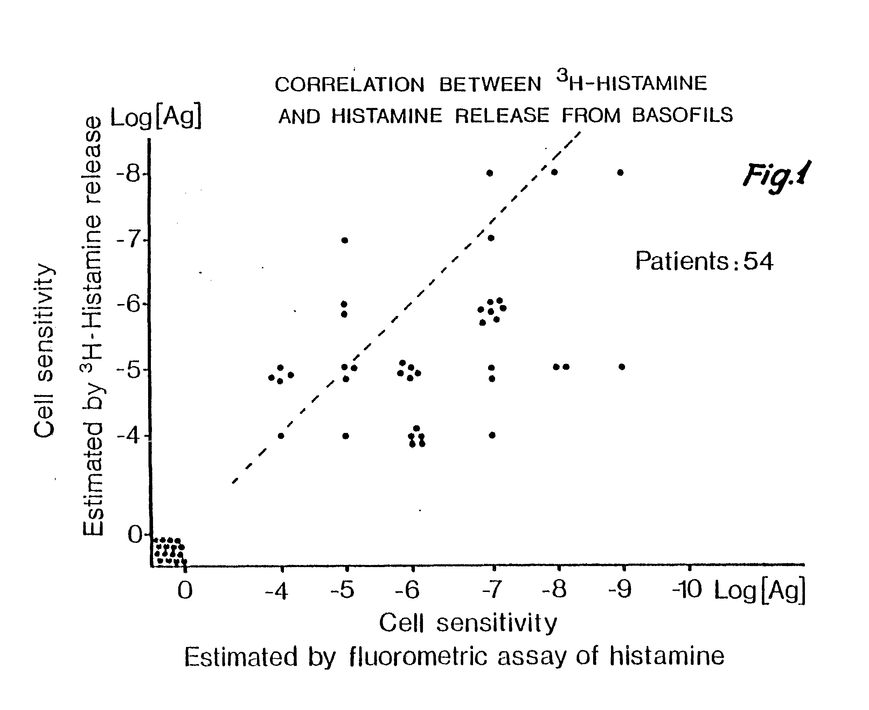

The measurements in Fig. 1 clearly confirm the lack of correlation between the method of Ref. 5 and the conventional fluorometric determination. The axes show declining concentrations (dilutions) of grass pollen antigen.

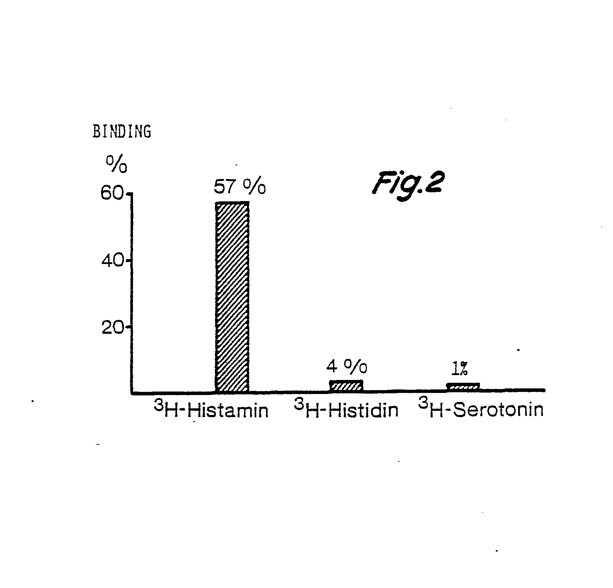

It is clearly apparent from Fig. 2 that histamine is bound surprisingly better than the low molecular compounds histidine and serotonin (5-hydroxy tryptamine). This is also of great importance to the reliability of the histamine determination since, in a number of the known methods, both compounds can interfere with histamine.

It is apparent from Fig. 3, which is discussed in greater detail below, that the two histamine metabolites 1,4-MEIAA and 1,5-MEIAA are not bound to glass microfibres in the same concentration range as histamine, as they are not able to displace bound labelled histamine from the fibres in a competitive binding assay.

Example 1
Preparation of glass fibre prepared tubes

"Whatman® GF/B" glass fibre filters are cut into lengths of about 1/2 mm. 3.4 g are mixed with 500 ml of redistilled water and homogenized for 2 minutes in an "ULTRA TURRAX" blender. The crushed fibres are left for 2 hours at room temperature for sedimentation. The heavy fibres (the longest ones) precipitate, and a supernatant clearly separated from the heavy fibres and containing suspended fibres of dimensions from about 20 µm to 2 µm is removed (a total of about 100 ml) and 100 µl of this supernatant are transferred to each plastic tube. The tubes are dried in an oven at 150°C (for 1 week), and are then taken out of the oven and are ready for use after cooling to about 20°C. With this drying procedure the glass fibres are fixed to the bottom of the tubes. The glass fibre-prepared tubes have unlimited shelf life.

Plotting of standard curve for histamine determination

Known dilutions of unlabelled histamine are prepared in Tris-AMC buffer (tris-(hydroxymethyl)-amino methane 25 mM, pH 7.6, NaCl 0.12 M, KCl 5 mM, $CaCl_2$ 0.6 mM, $MgCl_2$ 1.1 mM, human serum albumin 0.3 mg/ml and glucose 1 mg/ml). The histamine content was 25 ng, 50 ng, 100 ng, 200 ng, 400 ng and 800 ng/ml, respectively, and the 0-sample used was histamine-free buffer. These concentrations are used for the plotting of a standard curve. 100 µl each of these dilutions are transferred to each glass fibre prepared test tube, to which are added 10 µl of radio-actively labelled histamine ($2,5 - ^3H$ - histamine - dihydrochloride: $1.85.10^{-7}$ $ns^{-1}$ $ml^{-1}$ (500 nCi/ml) corresponding to $1.85.10^{-9}$ $ns^{-1}$/ sample (5 nCi/sample), specific activity about $1.961.10^{-8}$ $s^{-1}$/mmole (53 Ci/mmole)). The samples are incubated for 40 min at 37°C. To obtain uniform results it is essential to observe the same periods of time for each sample. The samples are washed for 15 s with redistilled water in a cell harvester (Tech-Nunc, Roskilde, Denmark). The residual water is discarded, and 1.2 ml of Filter Count (Packard) are added. The samples are counted for 1 min. in a liquid scintillation counter, the emission of β-radiation being recorded in counts per minutes (cpm). The sample without unlabelled histamine (0-binding) contains bound labelled histamine in an amount of typically 2000 cpm, which constitutes about 60% of the total labelled histamine added to the sample. The sample containing 25 ng/ml of unlabelled histamine binds to the fibres in an amount corresponding to about 15% of the 0-binding and thus gives a count of about 1700 cpm. Increasing amounts of histamine result in a corresponding proportional decrease in cpm— linearly up to 100 µg histamine/ml.

The standard curve shown in Fig. 3 with the symbol GF/B for two parallel tests shows a semilogarithmically linear correlation between % binding, expressed as cpm (the y-axis) and histamine content (the x-axis: logarithmic). The sensitivity of the assay is about 25 ng histamine/ml. The figure shows two corresponding tests with fibres of the type "Whatman® GF/C" prepared in the same manner, and these fibres exhibit a somewhat poorer correlation.

Example 2
Determination of specific allergy to grass pollen in 12 asthmatic patients by in vitro provocation of the basophil leucocytes of the patients with grass pollen was performed.

10 ml of blood are drawn from each patient by venipuncture. The blood is mixed with 0.5 ml of 0.2 M EDTA (pH 7.2). The sample is divided into two parts, one of which is analyzed as described in Ref. 2 to demonstrate the correlation between the method of the invention and fluorometric determination. 5 ml of blood are mixed with 1 ml of dextran (molecular weight 500,000 g/mol; 45 mg dissolved in 1 ml of 0.9% NaCl) to remove the erythrocytes (the red blood cells). The sample is carefully inverted and left for 30 min at room temperature. The sedimentation of the erythrocytes is more rapid than that of the leucocytes (the white blood cells). The plasma layer containing the leucocytes is transferred to another tube and suspended in 20 ml of Tris-AMC buffer (cf. Example 1). The leucocyte suspension is centrifuged for 10 min at 100 G and 16°C. The supernatant plasma is removed and discarded and the cells are suspended in 20 ml of Tris-AMC buffer and centrifuged for 10 min at 60 G and 16°C. The supernatant is again removed, and the cells are resuspended in 5 ml of Tris-AMC buffer. The cell suspension contains 2 to 4% of basophil leucocytes.

100 µl of this cell suspension are transferred to tubes prepared with glass microfibres as in Example 1. 10 µl of grass pollen are added in a 10-fold dilution series ($10^{-3}$, $10^{-4}$, $10^{-5}$ v/v) of a grass pollen standard preparation and 10 µl of isotope-labelled (tritiated) histamine are added to the tubes, corresponding to $1.85.10^{-9}$ s$^{-1}$/sample (5 nCi per sample). The samples are incubated for 40 min at 37°C. The samples are washed for 15 s with redistilled water in a cell harvester (Tech-Nunc, Roskilde, Denmark).

The residual water is discarded, and 1.2 ml of Filter Count (Packard) are added. The samples are counted in a liquid scintillation counter. In case of allergy to grass pollen, the basophil cells in the sample will release histamine in increasing amounts at increasing grass pollen concentrations. The released histamine is bound to the glass microfibre tubes in competition with the isotope-labelled histamine, and the binding of the isotope-labelled histamine will therefore decrease. A fall in the binding of the isotope-labelled histamine of 10% in relation to a cell sample without grass pollen is considered positive allergy to grass.

The histamine content in the unknown sample may also be calculated by the standard curve discussed in Example 1.

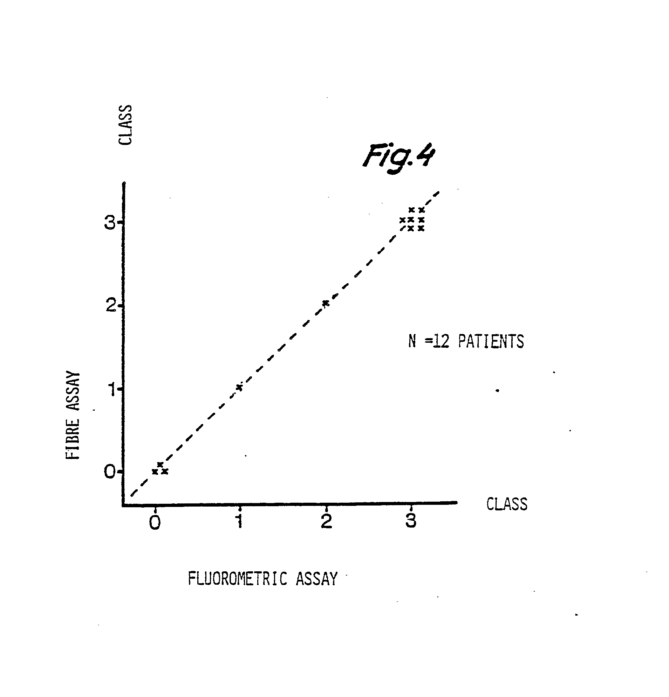

As appears from Fig. 4, an extremely good correlation was found between the histamine release measured by the glass microfibre method and fluorometric determination of histamine, since the investigation of 12 patients showed identical values of the histamine release measured by the two methods. It is noted that the sensitivity of the patient to the allergen is divided into classes where 0 means no reaction, while 3 represents the greatest reaction. Thus, the correlation applies to both non-allergic patients and all three classes of allergy.

Example 3
In a modification of the method according to the foregoing Example 2, histamine is determined directly after binding to glass microfibre filters.

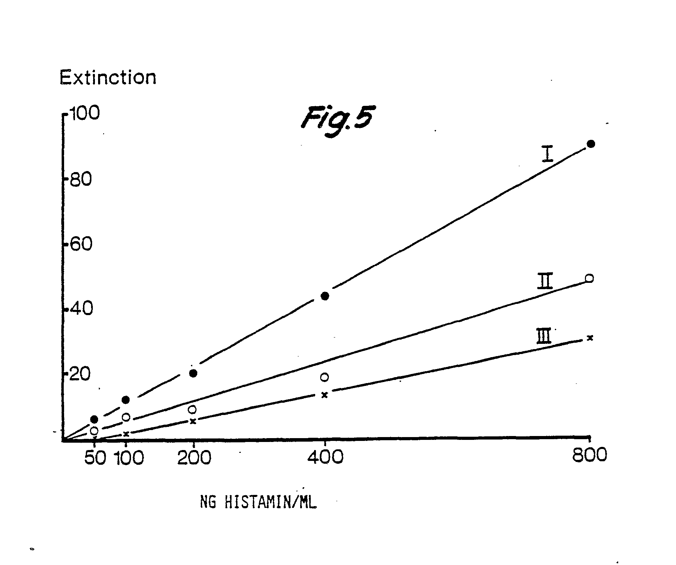

A: 10 µl of unlabelled histamine dissolved in Tris-AMC buffer, cf. Example 1 (800 ng—400 ng—200 ng—100 ng—50 ng and 0 histamine/ml Tris-AMC), are incubated with 6 mm diameter glass microfibre discs for 40 min at 37°C, followed by washing with $H_2O$ for 15 s in a cell harvester. Residual $H_2O$ is removed from the glass microfibre discs. Histamine bound to the discs is determined fluorometrically. Coupling is accomplished with 400 µl of NaOH/OPT (10 mg 0-phthaldialdehyde (Fluka) dissolved in 5 ml of methanol and mixed with 18 ml of 0.05 M NaOH) for 4 min at room temperature. To stabilise the fluorophore 400 µl of 0.175 M $H_3PO_4$ is added. The samples are centrifuged at 2800 g for 10 min. Reading of the extinction is performed using an AMINCO fluorometer (see curve III in Fig. 5).

B: The following control is included: 10 µl of histamine in the same concentrations as used in A are incubated with the discs for 40 min at 37°C. The samples are not washed in a cell harvester, but are immediately coupled as stated in A (see curve II in Fig. 5).

C: The following additional control is included: 10 µl of histamine in the same concentrations as used in A are incubated for 40 min at 37°C in the absence of the discs. The samples are not washed in a cell harvester, but are immediately coupled as stated in A (see curve I in Fig. 3).

The three curves thus show:

I: total amount of histamine added to the test tubes.

II: total amount of histamine added to the filter discs in the test tubes.

III: histamine binding to the filter discs after washing.

Although this procedure is not optimized in the above examples, curve III clearly shows the fine linear correlation between the various histamine concentrations and the binding to the fibres.

Example 4
In a preferred embodiment a microtiter plate is used as a means for carrying out a direct histamine determination according to the invention.

5 ml of blood is freed of plasma and erythrocytes in accordance with example 2. A glass microtiter plate with 96 wells which has been prepared with 100 µl of glass microfiber suspensions and dried in accordance with example 1 was used for the detection of the histamine content as a result of allergic reactions. 10 µl samples of the test allergens in three concentrations are added to the wells. 100 µl portions of the blood sample are added to the wells and the plate is left at room temperature for 20 min. During this incubation released histamine will bind to the glass microfibres. The microtiter plate is washed with water for 30 sec., following which the plate is ready for fluorometric assay.

100 µl NaOH/o-phthalaldehyde is added to each well in the microtiter plate and the plate is left at room temperature for 10 min., following which the reaction is stopped by adding 100 μl $H_3PO_4$ to each well.

The individual samples are then read in a fluorometer.

References

1. W. Lorenz et al.: A sensitive and specific method for the determination of histamine in human whole blood and plasma. Hoppe-Seyler's Z. Physiol. Chem. 353: 911—920, 1972.
2. P. Stahl Skov & S. Norn: A simplified method for measuring basophil histamine release and blocking antibodies in hay fever patients. Basophil histamine content and cell preservation. Acta Allergol. 32: 170—182, 1977.
3. R. P. Siraganian: Automated histamine release. A method for in vitro allergy diagnosis. Int. Archs Allergy appl. Immun. 49: 108—110, 1975.
4. K. M. Taylor et al.: An enzymatic-isotopic microassay for measuring allergic release of histamine from blood and mast cells in vitro. Int. Archs Allergy appl. Immun. 61: 19—27, 1980.
5. P. Stahl Skov et al.: $^3$H-histamine release from human leucocytes. Allergy 34: 261—263, 1979.
6. H. A. Bosse & O. Wassermann: Über eine Adsorption von Histamin an Glas. Med. Pharmacol. exp. 16: 459—461 (1967), (Chem. Abstr., Vol. 67 (1967) No. 62.688).
7. J. Murray & A. S. McGill: Effect of adsorption of histamine to glass surfaces on its estimation. J. Assoc. Off. Anal. Chem., Vol. 65, No. 1 (1982).

## Claims

1. A method for detecting or determining histamine in a body fluid, in which a sample of the body fluid is contacted with a binder for histamine and the amount bound is registered or measured, characterized by contacting the sample of fluid and optionally a predetermined amount of labelled histamine with borosilicate glass microfibres deposited onto a carrier in such a quantitative proportion between the glass microfibres and the fluid as will permit the histamine present to be bound to the microfibres; and registering or measuring the amount of histamine bound to the microfibres, without a previous desorption, either by a competitive determination based on a standard curve for known amounts of unlabelled histamine or by a direct determination.

2. The method according to claim 1 adapted for competitive histamine detection or determination, characterized by adding to a sample of body fluid possibly containing histamine prior to the contacting step, a predetermined amount of labelled histamine, registering the amount of labelled histamine bound to the glass microfibres, and comparing the registered amount with a standard curve based on competition with known amounts of unlabelled histamine.

3. The method according to claim 1 or 2 for detecting histamine possibly released by allergic reaction, characterized by adding a test allergen to a blood fraction from which the plasma has been removed, and registering or measuring the possibly released histamine bound to the fibres directly or competitively with labelled histamine.

4. The method according to claim 1, 2 or 3, characterized in that the glass microfibres used are in the form of fine particles having dimensions ranging from 2 to 20 μm.

5. The method according to any of claims 1 to 4, characterized in that the borosilicate glass microfibres used have the following oxide content:

|  | % |  | % |
|---|---|---|---|
| $SiO_2$ | 57.9 | CaO | 2.6 |
| $B_2O_3$ | 10.7 | MgO | 0.4 |
| $Fe_2O_3$ | 5.9 | BaO | 5.0 |
| $Al_2O_3$ }<br>$Na_2O$ } | 10.1 | ZnO | 3.9 |
|  |  | F | 0.6 |
| $K_2O$ | 2.9 |  |  |

6. The method according to any of claims 1 to 5 characterized in that the labelled histamine used is a radioactively labelled, fluorescent-labelled or enzyme-labelled histamine or histamine derivative.

7. An analytical means for use in the method of any of claims 1 to 6, characterized by comprising borosilicate glass microfibres deposited onto a carrier.

8. An analytical means according to claim 7 in which the carrier is a test tube, microtiter plate, foil or strip.

9. The analytical means according to claim 7 or 8 characterized in that the individual glass microfibres are as defined in claim 4 or 5.

10. An analytical means according to claim 7, 8 or 9 adapted for use in the detection or determination of histamine released by allergic reaction, characterized in further comprising at least one test allergen.

11. A diagnostic test device for use in the detection or determination of histamine in body fluids, which comprises an analytical means according to claim 7, 8, 9, or 10.

## Patentansprüche

1. Verfahren zum Nachweis oder zur Bestimmung von Histamin in Körperflüssigkeiten, bei dem eine Probe des Materials mit einem Bindungsmittel für Histamin in Kontakt gebracht und die gebundene Menge registriert oder gemessen wird, dadurch gekennzeichnet, dass eine Probe der Flüssigkeit und gegebenenfalls eine im voraus festgelegte Menge markierten Histamins mit auf einem Träger abgesetzten Borosilikatglasmikrofibern in solchen Mengenverhältnissen zwischen den Glasmikrofibern und der Flüssigkeit in Kontakt gebracht werden, die die Bindung des vorhandenen Histamins an die Mikrofibern erlauben, und dass die an die Mikrofibern gebundene Menge Histamin ohne vorhergehende Desorption entweder durch eine kompetitive Bestimmung auf Basis einer Standardkurve für bekannte Mengen unmarkierten Histamins oder durch eine direkte Bestimmung nachgewiesen oder bestimmt wird.

2. Verfahren nach Anspruch 1, bei dem eine kompetitive Histaminbestimmung vorgenommen wird, dadurch gekennzeichnet, dass eine im voraus festgelegte Menge markierten Histamins vor dem Kontakt mit den Glasmikrofibern der untersuchten gegebenenfalls histaminhaltigen Körperflüssigkeit zugesetzt und die an die Glasmikrofibern gebundene Menge markierten Histamins registriert und mit einer auf Konkurrenz mit bekannten Mengen unmarkierten Histamins basierten Standardkurve verglichen wird.

3. Verfahren nach Anspruch 1 oder 2 zum Nachweis von durch eine allergische Reaktion gegebenenfalls freigesetztem Histamin, dadurch gekennzeichnet, dass ein Testallergen einer Blutfraktion, aus der das Plasma entfernt worden ist, zugesetzt und das an die Fibern gebundene, gegebenenfalls freigesetzte Histamin dann direkt oder kompetitiv mit markiertem Histamin registriert wird.

4. Verfahren nach Anspruch 1, 2 oder 3, dadurch gekennzeichnet, dass die als Bindungsmittel verwendeten Glasmikrofibern in Form feiner Teilchen sind, die Dimensionen der Grössenordnung 2—20 µm aufweisen.

5. Verfahren nach Anspruch 1 bis 4, dadurch gekennzeichnet, dass die verwendeten Glasmikrofibern Borsilikatglasmikrofibern mit dem folgenden Oxidgehalt sind:

| | % | | % |
|---|---|---|---|
| $SiO_2$ | 57,9 | CaO | 2,6 |
| $B_2O_3$ | 10,7 | MgO | 0,4 |
| $Fe_2O_3$ | 5,9 | BaO | 5,0 |
| $Al_2O_3$ $Na_2O$ | 10,1 | ZnO | 3,9 |
| | | F | 0,6 |
| $K_2O$ | 2,9 | | |

6. Verfahren nach irgendwelchem der Ansprüche 1 bis 5, dadurch gekennzeichnet, dass als markiertes Histamin ein radioaktiv markiertes, fluoreszenmarkiertes oder enzymmarkiertes Histamin oder Histaminderivat verwendet wird.

7. Analytisches Mittel zur Verwendung bei der Durchführung des Verfahrens nach Anspruch 1 bis 6, dadurch gekennzeichnet, dass es auf einem Träger abgesetzte Borosilikatglasmikrofibern umfasst.

8. Analytisches Mittel nach Anspruch 7, dadurch gekennzeichnet, dass der Träger ein Reagenzglas, eine Mikrotiterplatte, Folie oder ein Streifen ist.

9. Analytisches Mittel nach Anspruch 7 oder 8, dadurch gekennzeichnet, dass die individuellen Glasmikrofibern wie im Anspruch 4 oder 5 definiert sind.

10. Analytisches Mittel nach Anspruch 7, 8 oder 9 zur Verwendung bei dem Nachweis oder der Bestimmung von durch allergische Reaktionen freigesetztem Histamin, dadurch gekennzeichnet, dass es weiterhin mindestens ein Testallergen umfasst.

11. Diagnostische Testausrüstung zur Verwendung bei dem Nachweis oder der Bestimmung von Histamin in Körperflüssigkeiten, dadurch gekennzeichnet, dass sie ein analytisches Mittel nach Anspruch 7, 8, 9 oder 10 umfasst.

**Revendications**

1. Une méthode pour détecter ou déterminer l'histamine dans un fluide corporel, dans laquelle un échantillon du fluide corporel est mis en contact avec un liant pour l'histamine et la quantité liée est enregistrée ou mesurée, caractérisée en ce qu'on met en contact l'échantillon de fluide et optionnellement une quantité prédéterminée d'histamine marquée avec des microfibres de verre de borosilicate déposées sur un véhicule en une proportion quantitative entre les microfibres de verre et le fluide telle qu'elle permettra à l'histamine présente de se lier aux microfibres, et en ce qu'on enregistre ou mesure la quantité d'histamine liée aux microfibres, sans désorption préalable, soit par une détermination concurrentielle basée sur une courbe standard pour des quantités connues d'histamine non marquée soit par une détermination directe.

2. La méthode selon la revendication 1 adaptée pour la détection ou la détermination concurrentielle de l'histamine, caractérisée en ce qu'on ajoute à un échantillon de fluide corporel contenant éventuellement de l'histamine avant l'étape de mise en contact, une quantité prédéterminée d'histamine marquée, en ce qu'on enregistre la quantité d'histamine marquée liée aux microfibres de verre, et en ce qu'on compare la quantité enregistrée avec une courbe standard basée sur la concurrence avec des quantités connues d'histamine non marquée.

3. La méthode selon la revendication 1 ou 2 pour détecter l'histamine éventuellement libérée par une réaction allergique, caractérisée en ce qu'on enregistre ou mesure l'histamine éventuellement libérée liée aux fibres directement ou en concurrence avec l'histamine marquée.

4. La méthode selon les revendications 1, 2 ou 3, caractérisée en ce que les microfibres de verre utilisées ont la forme de fines particules ayant des dimensions variant de 2 à 20 µm.

5. La méthode selon l'une quelconque des revendications 1 à 4, caractérisée en ce que les microfibres de verre de borosilicate utilisées ont la teneur en oxyde suivante:

| | % | | % |
|---|---|---|---|
| $SiO_2$ | 57.9 | CaO | 2.6 |
| $B_2O_3$ | 10.7 | MgO | 0.4 |
| $Fe_2O_3$ | 5.9 | BaO | 5.0 |
| $Al_2O_3$ $\rbrace$ $Na_2O$ | 10.1 | ZnO | 3.9 |
| | | F | 0.6 |
| $K_2O$ | 2.9 | | |

6. La méthode selon l'une quelconque des revendications 1 à 5 caractérisée en ce que l'histamine marquée utilisée est de l'histamine ou un dérivé de l'histamine marqué radioactivement, marqué par fluorescence ou marqué par un enzyme.

7. Un moyen analytique pour l'utilisation dans la méthode de l'une quelconque des revendications 1 à 6, caractérisé en ce qu'il comprend des microfibres de verre de borosilicate déposées sur un véhicule.

8. Un moyen analytique selon la revendication 7 dans lequel le véhicule est un tube test, une plaque, une lame ou une bande de microtitrage.

9. Le moyen analytique selon la revendication 7 ou 8 caractérisé en ce que les microfibres de verre individuelles sont comme définies dans la revendication 4 ou 5.

10. Un moyen analytique selon la revendication 7, 8 ou 9, adapté pour l'utilisation dans la détection ou la détermination de l'histamine libérée par une réaction allergique, caractérisé de plus en ce qu'il comprend au moins un allergène d'essai.

11. Un dispositif de test de diagnostic pour l'utilisation dans la détection ou la détermination de l'histamine dans les fluides corporels, qui comprend un moyen analytique selon les revendications 7, 8, 9 ou 10.

CORRELATION BETWEEN ³H–HISTAMINE
AND HISTAMINE RELEASE FROM BASOFILS

*Fig.1*

Patients:54

Fig.2

BINDING %

Fig.3

Fig.4

N =12 PATIENTS

FLUOROMETRIC ASSAY

Extinction

Fig.5

NG HISTAMIN/ML